# EUROPEAN PATENT APPLICATION

(11) **EP 4 840 943 A1**
(43) Date of publication of application: **18.11.2026**
(21) Application number: 24917240.4
(22) Date of filing: 30.12.2024
(51) Int. Cl.: C12N 5/09, C12N 5/0783, C12N 13/00, C07K 14/74, C07K 16/28, C07K 14/54, A61K 35/17, A61P 35/00

(54) **FEEDER CELL FOR ACTIVATION AND AMPLIFICATION OF NK CELLS AND USE THEREOF**

(30) Priority: 11.01.2024 KR 20240004642
(71) Applicant: Samsung Life Public Welfare Foundation, Seoul 04348 (KR); Research & Business Foundation Sungkyunkwan University, Suwon-si, Gyeonggi-do 16419 (KR); Seoul National University R&DB Foundation, Seoul 08826 (KR)
(72) Inventor: CHO, Deok, Seoul 06351 (KR); KIM, Hyun Young, Seoul 06351 (KR); KIM, Jin Ho, Seoul 06351 (KR); JO, Yu Jung, Seoul 06351 (KR); LEE, Mi Jeong, Seoul 06351 (KR); HAN, Byung Woo, Seoul 08826 (KR); KANG, Jin Mo, Seoul 08826 (KR)
(74) Representative: Weickmann & Weickmann PartmbB
(86) International application number: PCT/KR2024/021499
(87) International publication number: WO 2025/150751

(57) **Abstract**

The present invention relates to feeder cells for activation and expansion of NK cells and use thereof, wherein the feeder cells for NK cells express one or more of mbCD16, mbIL-12, and mbIL-18 together with HLA-E, thereby stimulating NK cells to enhance the activation and expansion of NK cells, and such NK cells can be used as a cell therapy product and as a pharmaceutical composition for the prevention or treatment of cancer or infectious diseases.

## Description

### Technical Field

The present invention relates to feeder cells for activation and expansion of NK cells and use thereof.

### Background Art

Natural killer (NK) cells are one of the cytotoxic lymphocytes important for innate immunity, and primarily respond to virus-infected cells and cancer cells. Such responses are governed by signals derived from interactions between various activating receptors and inhibitory receptors on NK cells and their corresponding ligands on target cells. Normal cells express ligands for the inhibitory receptors of NK cells (e.g., MHC), thereby transmitting inhibitory signals, so that a defense mechanism against NK cells operates. In contrast, infected or abnormal cells express ligands for the activating receptors of NK cells, through which activating signals are transmitted, and the activating signals are further reinforced by stimuli such as cytokines, thereby breaking the balance with inhibitory signals and triggering NK cell attack. NK cells secrete perforin to perforate the cell membrane of infected cells or cancer cells, and release granzymes to activate apoptotic signals in those cells, thereby inducing cell death.

NK cells, which have the ability to directly recognize and destroy cancer cells, have recently been the subject of diverse research and clinical trials as one of anti-cancer therapeutic strategies. In particular, in addition to the characteristics of conventional NK cells, adaptive NK cells, which retain memory of responses to stimuli such as tumors or infections, have begun to attract attention. Adaptive NK cells display increased expression of the NKG2C activating receptor following HCMV infection and exhibit characteristics similar to memory cells, such as long-term survival. Research is ongoing to apply these adaptive NK cells to cancer treatment to compensate for the limitations of conventional NK cells.

Although the clinical significance of adaptive NK cells is being emphasized, and despite studies demonstrating their expansibility (Int. J. Mol. Sci. 2022, 23, 9426), they are still insufficient in terms of the features characteristic of adaptive NK cells - namely, reduction of FcεRIγ and enhancement of ADCC efficacy. Therefore, to effectively utilize adaptive NK cells in cancer treatment, it is necessary to develop a selective expansion technology that focuses on securing a sufficient cell number of adaptive NK cells so as to maximize their adaptive characteristics.

### [Prior Art Documents]

### [Patent Documents]

KR Patent No. 10-1525199
US Patent No. 10,995,317

### DISCLOSURE OF THE INVENTION

### Technical Problem

An object of the present invention is to provide feeder cells for activation and expansion of NK cells.

Another object of the present invention is to provide a composition for activation and expansion of NK cells comprising the feeder cells.

Another object of the present invention is to provide a method for activation and expansion of NK cells using the feeder cells or the composition.

Another object of the present invention is to provide a method for assaying the activity of NK cells using the feeder cells or the composition.

Another object of the present invention is to provide a method for providing information for diagnosis of NK cell-related diseases using the feeder cells or the composition.

Another object of the present invention is to provide NK cells produced by the method for activation and expansion of NK cells.

Another object of the present invention is to provide a cell therapy product comprising the NK cells as an active ingredient.

Another object of the present invention is to provide a pharmaceutical composition for the prevention or treatment of cancer or infectious diseases, comprising the NK cells as an active ingredient.

Another object of the present invention is to provide a use of the NK cells.

Another object of the present invention is to provide a method for treating cancer or infectious diseases using the NK cells.

### Technical Solution

### Feeder Cells for Activation and Expansion of NK Cells

One aspect of the present invention provides feeder cells for activation and expansion of NK cells, which express human leukocyte antigen E (HLA-E); and one or more selected from the group consisting of a membrane-bound anti-CD16 antibody, membrane-bound interleukin-12, and membrane-bound interleukin-18.

The term "feeder cell" as used in the present invention refers to a cell that, while incapable of mitosis, is metabolically active and thus produces various metabolites that assist in the proliferation of target cells; such cells are used after treatment with mitomycin C or irradiation with radiation such as X-rays or γ-rays to inactivate cell division. The feeder cells in the present invention are intended for the activation and expansion of NK cells, and any animal-derived cell known in the art may be used without limitation.

According to one embodiment of the present invention, the feeder cells may be selected from the group consisting of K562, RPMI8866, ARH77, EBV_LCL, 721.221, and HFWT.

In general, K562, a representative feeder cell line used for NK cell proliferation, is used solely for NK cell proliferation and therefore must not proliferate itself. Accordingly, prior to co-culture with NK cells, the K562 cell line is pretreated by irradiation with high-dose radiation (e.g., 50 to 100 Gy), so that the K562 cell line itself does not proliferate at all while only assisting in NK cell proliferation. Meanwhile, for efficient NK cell proliferation, most cytokines such as IL-2 and IL-15 must be continuously exposed to NK cells. Therefore, when cancer cell line-based feeder cells are genetically engineered to express cytokines such as IL-2 and/or IL-15, there is a problem that the selective proliferation efficiency of NK cells is reduced because the irradiation-pretreated cancer cell lines cannot survive for extended periods during culture. However, the feeder cells for activation and expansion of NK cells according to the present invention can selectively and more efficiently obtain NK cells by being genetically engineered to express human leukocyte antigens.

According to one embodiment of the present invention, the NK cells may be adaptive NK cells.

The term "NK cell" or "natural killer cell" as used in the present invention refers to cytotoxic lymphocytes constituting a major component of the innate immune system, defined as large granular lymphocytes (LGL) and constituting a third cell type differentiated from common lymphoid progenitor (CLP)-generated B and T lymphocytes. In NK cells, CD16 (FcγRIII) is a distinctive surface protein, and CD56 is also a major surface protein; however, CD3, which is expressed on T cells, is absent.

The term "adaptive NK cell" as used herein refers to a specialized NK cell capable of forming immunological memory, which is involved in the adaptive immune system. In addition, the adaptive NK cells do not have antigen specificity, and represent a cell subgroup that survives longer in the body than other NK cells and exhibits stronger antibody-dependent cell-mediated cytotoxicity (ADCC) and the like. For example, the NK cells or adaptive NK cells may be, but are not limited to, NKG2C+ NK cells and FcεRγ-NK cells.

The term "human leukocyte antigen (HLA)" as used herein refers to a cell membrane glycoprotein molecule expressed on the surface of nucleated cells in humans, which presents antigens to T lymphocytes to elicit adaptive immunity against invading antigens and also protects normal cells from killing by NK cells. The HLA may be HLA-A, HLA-B, HLA-C, HLA-E, HLA-F, HLA-G, or the like.

According to one embodiment of the present invention, the human leukocyte antigen E (HLA-E) may comprise the nucleic acid sequence of SEQ ID NO: 1.

Specifically, the HLA-E may have at least about 70%, at least about 75%, at least about 80%, at least about 85%, at least about 90%, at least about 92%, at least about 95%, at least about 97%, at least about 98%, or at least about 99% sequence homology with the nucleic acid sequence of SEQ ID NO: 1 or the amino acid sequence thereof.

The feeder cells for activation and expansion of NK cells according to the present invention are genetically engineered cells or cell lines that are genetically engineered to express HLA-E together with one or more of a membrane-bound anti-CD16 antibody, membrane-bound IL-12, and membrane-bound IL-18 on the cell membrane. The term "genetic engineering" or "genetically engineered" herein refers to the act of introducing one or more genetic modifications into a cell or a cell produced thereby.

Specifically, the feeder cells may comprise an exogenous gene encoding HLA-E; and one or more selected from the group consisting of a membrane-bound anti-CD16 antibody, membrane-bound IL-12, and membrane-bound IL-18. The term "exogenous" herein means that the referenced molecule or referenced activity has been introduced into a host cell. The molecule may be introduced into the host genetic material, for example by insertion into the host chromosome, or introduced as non-chromosomal genetic material such as a plasmid. In the context of expression of a coding nucleic acid, the term "exogenous" indicates that the coding nucleic acid has been introduced into the individual in a form capable of expression. In the context of a biosynthetic activity, the term "exogenous" indicates an activity that has been introduced into a host parent cell. The source may be, for example, a homologous or heterologous coding nucleic acid that, after introduction into the host parent cell, expresses the referenced activity. Conversely, "endogenous" refers to the referenced molecule or activity that is present in the host cell. Similarly, in the context of expression of a coding nucleic acid, the term "endogenous" refers to the expression of a coding nucleic acid contained within an individual. The term "heterologous" refers to a molecule or activity from a source other than the referenced species, and "homologous" refers to a molecule or activity derived from the host parent cell. Accordingly, exogenous expression of a coding nucleic acid may utilize either or both heterologous and homologous coding nucleic acids.

According to one embodiment of the present invention, the feeder cells may comprise a nucleic acid encoding HLA-E; and one or more selected from the group consisting of a membrane-bound anti-CD16 antibody, membrane-bound IL-12, and membrane-bound IL-18.

In addition, according to one embodiment of the present invention, the feeder cells may be transformed with a vector comprising a nucleic acid encoding HLA-E; and one or more selected from the group consisting of a membrane-bound anti-CD16 antibody, membrane-bound IL-12, and membrane-bound IL-18.

The vector refers to a genetic construct comprising regulatory elements operably linked such that a gene insert is expressed, which is capable of expressing a target protein in an appropriate host cell. The vector may comprise expression regulatory elements such as a promoter, operator, initiation codon, termination codon, polyadenylation signal, and/or enhancer, and the promoter of the vector may be constitutive or inducible. Also, the vector may be an expression vector capable of stably expressing the target protein or fusion proteins thereof in a host cell. The expression vector may be a conventional one used in the art for expressing foreign proteins in plants, animals, or microorganisms. The recombinant vector can be constructed by various methods known in the art. For example, the vector may comprise a selection marker for selecting host cells containing the vector, and, when it is a replicable vector, it may comprise a replication origin. In addition, the vector may self-replicate or be integrated into the host DNA, and may be selected from the group consisting of plasmids, lentiviruses, adenoviruses, adeno-associated viruses, retroviruses, herpes simplex viruses, and vaccinia viruses.

Also, in the vector, a polynucleotide sequence encoding the target protein or fusion protein described above may be operably linked to a promoter. The term "operably linked" as used in the present invention refers to a functional linkage between a nucleic acid expression regulatory sequence (e.g., an array of promoter, signal sequence, or transcriptional regulator binding sites) and another nucleic acid sequence, whereby the regulatory sequence controls the transcription and/or translation of the other nucleic acid sequence.

The term "membrane-bound anti-CD16 antibody (mbCD16)" as used in the present invention refers to an anti-CD16 antibody bound to the cell membrane, and may be distinguished from an anti-CD16 antibody secreted extracellularly.

According to one embodiment of the present invention, the membrane-bound anti-CD16 antibody may be one or more selected from the group consisting of monoclonal antibodies, polyclonal antibodies, recombinant antibodies, and fragments thereof.

The anti-CD16 antibody may include any antibody or fragment thereof having a structure and function that specifically binds to the antigen, i.e., CD16, and exhibits reactivity thereto. The term "antibody" herein refers to a specific protein molecule directed against an antigenic site. In the present invention, it refers to an antibody that specifically binds to each protein, and includes monoclonal antibodies, polyclonal antibodies, antibodies having a full-length chain structure (full-length antibodies), and recombinant antibodies. The term "specifically binds" means that the binding affinity for the target substance is superior to that for other substances to an extent sufficient to detect the presence or absence of the target substance by the binding. In addition, the antibody includes not only a complete form having two full-length light chains and two full-length heavy chains, but also functional fragments of antibody molecules. The functional fragments of antibody molecules refer to fragments retaining at least the antigen-binding function, and may be Fab, F(ab'), F(ab')2, or Fv.

The membrane-bound anti-CD16 antibody according to the present invention is preferably a monoclonal antibody having a single affinity that binds to only one antigenic determinant (antigenic determinant or epitope).

The membrane-bound anti-CD16 antibody may be, as monoclonal antibody clones, eBioCB16 (CB16), 3G8, B73.1, MEM-154, or the like, but is not limited thereto.

According to one embodiment of the present invention, the monoclonal antibody may be one or more selected from the group consisting of CB16, 3G8, B73.1, and MEM-154.

The epitopes to which these monoclonal antibodies bind may partially overlap or may differ from each other.

The term "membrane-bound interleukin (mbIL)" as used in the present invention refers to an interleukin bound to the cell membrane, and may be distinguished from an interleukin secreted extracellularly. mbIL-12 may have at least about 70%, at least about 75%, at least about 80%, at least about 85%, at least about 90%, at least about 92%, at least about 95%, at least about 97%, at least about 98%, or at least about 99% sequence homology with the nucleic acid sequence of SEQ ID NO: 11 or the amino acid sequence thereof. mbIL-18 may have at least about 70%, at least about 75%, at least about 80%, at least about 85%, at least about 90%, at least about 92%, at least about 95%, at least about 97%, at least about 98%, or at least about 99% sequence homology with the nucleic acid sequence of SEQ ID NO: 12 or the amino acid sequence thereof.

According to one embodiment of the present invention, the feeder cells may express human leukocyte antigen E (HLA-E) and membrane-bound anti-CD16 antibody (mbCD16).

In addition, according to one embodiment of the present invention, the feeder cells may express human leukocyte antigen E (HLA-E), membrane-bound interleukin-12 (mbIL-12), and membrane-bound interleukin-18 (mbIL-18).

In addition, according to one embodiment of the present invention, the feeder cells may express human leukocyte antigen E (HLA-E), membrane-bound anti-CD16 antibody (mbCD16), membrane-bound interleukin-12 (mbIL-12), and membrane-bound interleukin-18 (mbIL-18).

### Composition for Activation and Expansion of NK Cells

One aspect of the present invention provides a composition for activation and expansion of NK cells comprising the feeder cells.

The specific details of the feeder cells are as described above.

The composition for activation and expansion of NK cells according to the present invention comprises feeder cells expressing one or more selected from the group consisting of a membrane-bound anti-CD16 antibody, membrane-bound IL-12, and membrane-bound IL-18 together with HLA-E, thereby being capable of inducing expansion and/or activation of NK cells, and thus being usefully employed in the culture, isolation, or proliferation of NK cells.

The NK cells in the present invention include NK cells derived from any tissue source without additional modification, and may include not only mature NK cells but also NK progenitor cells. The NK cells are activated in response to interferons or macrophage-derived cytokines, and comprise two types of surface receptors - activating receptors and inhibitory receptors - that control the cytotoxic activity of the cells. The NK cells may be produced from hematopoietic cells, such as hematopoietic stem cell or progenitor cells, derived from any source, for example, placental tissue, placental perfusate, cord blood, placental blood, peripheral blood, spleen, liver, and the like.

According to one embodiment of the present invention, the composition may further comprise a cytokine for activation of NK cells.

The term "cytokine" as used in the present invention refers to a protein (5 to 20 kDa) that plays a role in cell signaling, is released by cells, and affects the behavior of the cells releasing the cytokines and/or other cells. The cytokines can be classified functionally, and examples thereof include interleukins (IL), lymphokines, monokines, chemokines, interferons (IFN), hematopoietic factors, growth factors, tumor necrosis factor (TNF) superfamily, adipokines, and neurotrophic factors. The cytokines may be produced by a wide range of cells, including immune cells such as macrophages, B lymphocytes, T lymphocytes, mast cells, and monocytes, endothelial cells, fibroblasts, and stromal cells, but are not limited thereto. Also, the cytokines may be produced by more than one type of cell. The cytokines act through receptors and are particularly important in the immune system, regulating the balance between humoral and cell-mediated immune responses, and regulating the maturation, growth, and responsiveness of cell populations.

The cytokines of the present specification may be naturally occurring cytokines or may be mutated versions of naturally occurring cytokines. The term "naturally occurring" may also be referred to as wild-type and includes allelic variants. The "mutated version of a naturally occurring cytokine" or "mutation" refers to specific mutations made to the naturally occurring sequence in order to alter the function, activity, and/or specificity of the cytokine. For example, mutations may enhance or reduce the function, activity, and/or specificity of the cytokine. Mutations may include deletions or additions of one or more amino acid residues of the cytokine.

The cytokines may be one or more selected from the group consisting of BMP (Bone morphogenetic protein) family, CCL (Chemokine ligands) family, CMTM (CKLF-like MARVEL transmembrane domain containing member) family, CXCL (C-X-C motif ligand) family, GDF (Growth/differentiation factor) family, growth hormone, IFN family, IL family, TNF family, GPI (glycophosphatidylinositol), SLUPR-1 (Secreted Ly-6/uPAR-Related Protein 1), SLUPR-2 (Secreted Ly-6/uPAR-Related Protein 2), and combinations thereof.

According to one embodiment of the present invention, the composition may further comprise an interleukin or a mutant thereof as the cytokine.

A number of interleukins are synthesized by monocytes, macrophages, and endothelial cells as well as helper CD4 T lymphocytes, and can promote the development and differentiation of T and B lymphocytes and hematopoietic cells.

More specifically, the interleukin may further comprise one or more selected from the group consisting of IL-1, IL-2, IL-3, IL-4, IL-5, IL-6, IL-7, IL-8 (CXCL8), IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-35, and IL-36, but is not limited thereto.

### Method for Activation and Expansion of NK Cells

One aspect of the present invention provides a method for activation and expansion of NK cells, comprising the step of contacting NK cells with the feeder cells or a composition for activation and expansion of NK cells comprising the feeder cells.

The specific details of the feeder cells and the composition for activation and expansion of NK cells comprising the feeder cells are as described above.

The method for activation and expansion of NK cells of the present invention is a process of obtaining a sample comprising NK cells from a subject, then mixing NK cells or a population thereof with the feeder cells or the feeder cells included in the composition for co-culture (co-culturing), thereby stimulating, activating, or expanding a subpopulation of NK cells.

The sample may be a biological sample derived from a subject, for example, a mammal including a human. Also, the biological sample may be isolated from a subject, and may be, for example, blood, whole blood, serum, plasma, lymph fluid, urine, feces, tissue, cells, organs, bone marrow, saliva, sputum, cerebrospinal fluid, or a combination thereof, but is not limited thereto.

According to one embodiment of the present invention, the NK cells may be isolated from a blood sample, and for example may be whole blood.

The whole blood includes peripheral blood mononuclear cells (PBMC), purified NK cells, primary resting cells (i.e., cells isolated directly from blood), and the like.

The term "stimulation of NK cells" as used in the present invention may mean increasing the activity, for example, cytotoxic activity, of NK cells *in vitro* or *in vivo*, or generating, increasing, expanding, or proliferating activated NK cells.

According to one embodiment of the present invention, the step may be co-culturing NK cells and feeder cells.

Specifically, the NK cells may be derived from PBMCs, and the feeder cells may be separately treated to inactivate their proliferation.

According to one embodiment of the present invention, the feeder cells may be irradiated with 50 Gy to 300 Gy of radiation.

According to one embodiment of the present invention, the co-culture may involve culturing 0.1× 10⁶ to 10 × 10⁶ feeder cells per 1 × 10⁶ NK cells.

According to one embodiment of the present invention, the co-culture may be performed in the presence of a cytokine.

Specifically, the cytokine may be an interleukin or a mutant thereof, and the interleukin may further comprise one or more selected from the group consisting of IL-1, IL-2, IL-3, IL-4, IL-5, IL-6, IL-7, IL-8 (CXCL8), IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-35, and IL-36.

For example, the cytokines added to the co-culture may be IL-2 and IL-15. In detail, the co-culture may comprise culturing in the presence of IL-2 while adding IL-15 to the culture medium. The IL-2 may be used at a concentration of 1 to 500 U/mL, 1 to 400 U/mL, 1 to 300 U/mL, 1 to 200 U/mL, 1 to 100 U/mL, 1 to 50 U/mL, or 5 to 30 U/mL. The IL-15 may be used at a concentration of 1 to 50 ng/mL, 1 to 40 ng/mL, 1 to 30 ng/mL, 1 to 20 ng/mL, or 1 to 10 ng/mL.

According to one embodiment of the present invention, the co-culture may be performed for 2 to 30 days.

NK cells for which co-culture according to the present invention has been completed may be in an activated state. Activated NK cells may refer to cells in which the cytotoxicity, or the innate immunoregulatory capacity of NK cells, has been activated compared to parent cells, for example hematopoietic cells, or NK progenitor cells. The activated NK cells or a population enriched with activated NK cells may be evaluated by detecting at least one functionally relevant marker, for example, CD16, CD57, CD69, CD94, CD161, CD158a, CD158b, NKp30, NKp44, NKp46, DNAM-1, 2B4, KIR (e.g., KIR2DL1, KIR2DL2/3, KIR3DL1), and members of the NKG2 family of activating receptors (e.g., NKG2A, NKG2C, NKG2D).

The method for activation and expansion of NK cells according to the present invention may further comprise, after the step of contacting the NK cells with the feeder cells or the composition for activation and expansion of NK cells comprising the feeder cells, a step of recovering the activated NK cells.

The recovery of the NK cells may be performed using any cell recovery method known in the art without limitation.

### Method for Assaying NK Cell Activity, and Method for Providing Information for Diagnosis of NK Cell-Related Diseases

One aspect of the present invention provides a method for assaying NK cell activity, comprising steps of: i) contacting NK cells with the feeder cells or the composition to activate the NK cells; and ii) analyzing the degree of activation of the activated NK cells.

Also, one aspect of the present invention provides a method for providing information for diagnosis of NK cell-related diseases, comprising steps of: i) contacting NK cells with the feeder cells or the composition to activate the NK cells; and ii) analyzing the degree of activation of the activated NK cells.

The description of step i) overlaps with the method for activation and expansion of NK cells described above, and the specific details of the feeder cells and the composition are as described above.

In the method for assaying NK cell activity of the present invention, the NK cells in step i) may coexist with other blood cells or lymphocytes, and, if necessary, may be isolated from a blood sample before stimulation. For example, when NK cells coexist with other blood cells or lymphocytes, the isolation step may take place after stimulation; and when using only NK cells as lymphocytes, stimulation may take place after the isolation step. The purity and composition of the isolated NK cells may vary to the extent required for the experiment, without particular limitation as known in the art. The NK cells may be used as purified from the sample if necessary, or may be proliferated to secure conditions or cell numbers appropriate for the experiment before use.

However, in the case of certain combinations of factors that are specific to NK cells, when a combination of factors specific to the NK cells is used in the method of the present invention, the step of isolating the NK cells may not be essential.

Step ii) involves measuring the degree of activation of NK cells by a method known in the art and comparing it with a normal control.

According to one embodiment of the present invention, the degree of activation of the NK cells in step ii) may be measured as one or more selected from the group consisting of degranulation activity, cytotoxicity activity, and cytokines secreted upon NK cell stimulation.

The degranulation activity may refer to, for example, induction of lysis of target cells by perforin or granzyme secretion, and may be analyzed using FACS. Specifically, a method may be used in which PBMCs isolated from whole blood samples or purified NK cells are stimulated, and then CD107a expression, which is proportional to degranulation, is measured using a fluorochrome-conjugated antibody.

The cytotoxicity activity can be measured, for example, by culturing with a culture comprising target cells capable of activating NK cells labeled with a europium fluorescent dye and then measuring the amount of fluorescent dye released through target cell lysis using a microplate reader.

The cytokines secreted upon NK cell stimulation may be one or more selected from the group consisting of IFN-γ, TNF-α, TNF-β, MIP1-α, MIP-1β, RANTES, IL-8, and IL-10. The expression analysis of such NK cell immunostimulatory factors may be performed using FACS, intracellular cytokine staining, or ELISA. Specifically, a method may be used in which NK cell surface is stained using a specific fluorochrome-conjugated antibody, the cells are then permeabilized, and the expression of immunostimulatory factors within NK cells is measured by staining the cytokines or the like with another specific fluorochrome-conjugated immunostimulatory factor antibody (e.g., anti-IFN-γ antibody).

According to one embodiment of the present invention, in step ii), the degree of activation of the activated NK cells may be compared with that of normal NK cells.

Specifically, when normal NK cells are stimulated with the aforementioned feeder cells under equivalent conditions as the test group using normal NK cells as a control, the case in which the activation phenomenon occurring in normal NK cells is significantly higher, absent, or significantly reduced in the test group is determined to be abnormal. Through such comparison, pathological signs of diseases associated with abnormal NK cells, viral infections, the presence of cancer cells, and specific cancers can be determined, and prognoses for such diseases can be predicted. The term "normal NK cells" refers to NK cells possessed by or derived from an individual that does not have a disease, wherein the individual not having the disease does not have at least physical, genetic, or extrinsic conditions known to affect NK cell activity.

In the method for providing information for diagnosis of NK cell-related diseases of the present invention, the NK cell-related disease is one that exhibits abnormal NK cell activity, and may be, for example, hypersensitivity immune disease, autoimmune disease, immune rejection, immunodeficiency disease, histiocytosis, cancer, type 2 diabetes, parasitic infectious disease, and viral disease. The hypersensitivity immune disease may be one or more selected from asthma and sinusitis; the autoimmune disease may be one or more selected from lupus, multiple sclerosis, type 1 diabetes, and rheumatoid arthritis; or the histiocytosis may be any one or more selected from HLH, XLP1, and XLP2. Hemophagocytic lymphohistiocytosis (HLH) may encompass the meaning of class 2 Langerhans cell histiocytosis, erythrophagocytic lymphohistiocytosis (familial, sporadic), infection-associated hemophagocytic syndrome, virus-associated hemophagocytic syndrome, histiocytosis with massive lymphadenopathy, or reticulohistiocytosis. The term "cancer" includes tumors, hematologic cancers, or solid tumors, and includes those that impair the synergistic activity of an individual's NK cells or that do not elicit the synergistic activity of NK cells as target cells under specific conditions. In one embodiment, the cancer may be selected from the group consisting of lung cancer, liver cancer, esophageal cancer, gastric cancer, colorectal cancer, small intestinal cancer, pancreatic cancer, melanoma, breast cancer, oral cancer, brain tumor, thyroid cancer, parathyroid cancer, renal cancer, cervical cancer, sarcoma, prostate cancer, urethral cancer, bladder cancer, testicular cancer, hematologic cancer, lymphoma, skin cancer, psoriasis, and fibroadenoma. In one embodiment, the cancer may be pancreatic cancer or B cell lymphoma. In one embodiment, the viral disease may be hepatitis B. In one embodiment, the immunodeficiency disease may be DiGeorge syndrome or Chediak-Higashi syndrome.

The information on NK cell activity-related diseases can be used to determine abnormal NK cells when activation of the test NK cells is not detected compared to normal NK cells, or to determine that the target cells are associated with a specific disease when NK cells that have lost activity against a specific receptor abnormally elicit or fail to elicit activity against the target cells.

### NK Cells, Cell Therapy Product, Pharmaceutical Composition, Use of NK Cells, and Method for Treating Diseases

One aspect of the present invention provides NK cells produced by the method for activation and expansion of NK cells.

Also, one aspect of the present invention provides a cell therapy product comprising the NK cells as an active ingredient.

Also, one aspect of the present invention provides a pharmaceutical composition for the prevention or treatment of cancer or infectious diseases, comprising the NK cells as an active ingredient.

Also, one aspect of the present invention provides a use of the NK cells for the manufacture of a medicament.

Also, one aspect of the present invention provides a method for treating cancer or infectious diseases, comprising the step of administering the NK cells to a subject.

According to one embodiment of the present invention, the NK cells may comprise NK cells or a population thereof.

The term "disease" as used in the present invention may refer to one pathological condition, in particular cancer, infectious disease, inflammatory disease, metabolic disease, autoimmune disorder, degenerative disease, cell death-related disease, and graft rejection.

The term "treatment" as used in the present invention refers to or encompasses the alleviation, inhibition of progression, or prevention of a disease, disorder, or condition, or one or more symptoms thereof, and "active ingredient" or "pharmaceutically effective amount" may mean any amount of a composition used in the course of carrying out the invention provided herein that is sufficient to alleviate, inhibit the progression of, or prevent a disease, disorder, or condition, or one or more symptoms thereof.

The terms "administering", "introducing", and "transplanting" as used in the present invention are used interchangeably and may refer to the placement of a composition according to one embodiment into a subject by a method or route that results in at least partial localization of the composition to a desired site. A composition according to one embodiment may be administered by any suitable route that delivers at least a portion of the cells or cellular components of the composition to a desired location within a living subject. The survival period of cells following administration to a subject may range from as short as a few hours, e.g., 24 hours, to several days, to as long as several years.

The term "isolated cells", e.g., "isolated immune cells", as used in the present invention refers to cells that are substantially separated from the tissue from which the cells originated, e.g., hematopoietic cells.

The method of administration of the pharmaceutical composition according to one embodiment is not particularly limited, but the composition may be administered parenterally, such as intravenously, subcutaneously, intraperitoneally, by inhalation, or by topical application, or may be administered orally depending on the intended method. The dosage varies in range depending on the patient's body weight, age, sex, health condition, diet, time of administration, method of administration, excretion rate, and severity of disease, and the like. The daily dosage refers to the amount of a therapeutic agent according to one aspect that is sufficient for treatment of an alleviated disease state when administered to a subject in need of treatment. The effective amount of a therapeutic agent varies depending on the specific compound, disease state and its severity, and the subject in need of treatment, and can be routinely determined by one skilled in the art. As a nonlimiting example, the dosage for humans of a composition according to one aspect may vary depending on the patient's age, body weight, sex, dosage form, health condition, and degree of disease. Based on an adult patient weighing 70 kg, for example, approximately 1,000 to 10,000 cells/dose, 1,000 to 100,000 cells/dose, 1,000 to 1,000,000 cells/dose, 1,000 to 10,000,000 cells/dose, 1,000 to 100,000,000 cells/dose, 1,000 to 1,000,000,000 cells/dose, or 1,000 to 10,000,000,000 cells/dose may be administered once to several times per day at regular time intervals, or may be administered multiple times at regular time intervals.

The term "subject" as used in the present invention refers to a subject in need of treatment for a disease, and more specifically refers to a human or a non-human primate, or mammals such as mice, rats, dogs, cats, horses, and cattle.

According to one embodiment of the present invention, the pharmaceutical composition may comprise a pharmaceutically acceptable carrier and/or additive. For example, it may comprise sterile water, physiological saline, conventional buffers (phosphoric acid, citric acid, or other organic acids), stabilizers, salts, antioxidants (such as ascorbic acid), surfactants, suspending agents, isotonicity agents, or preservatives. For topical administration, combinations with organic substances such as biopolymers, inorganic substances such as hydroxyapatite, and specifically collagen matrices, polylactic acid polymers or copolymers, polyethylene glycol polymers or copolymers, and chemical derivatives thereof may also be included. When the pharmaceutical composition is formulated in a form suitable for injection, immune cells or a substance that increases their activity may be dissolved in a pharmaceutically acceptable carrier or frozen in a dissolved solution.

The pharmaceutical composition according to one embodiment may appropriately comprise, if necessary depending on the method of administration or formulation, suspending agents, solubilizers, stabilizers, isotonicity agents, preservatives, adsorption inhibitors, surfactants, diluents, excipients, pH adjusters, analgesic agents, buffers, reducing agents, antioxidants, and the like. Pharmaceutically acceptable carriers and preparations suitable for the present invention including those exemplified above are described in detail in [Remington's Pharmaceutical Sciences, 19th ed., 1995]. The pharmaceutical composition according to one embodiment may be manufactured in unit dosage form or may be prepared by enclosing in a multi-dose container by formulating it using a pharmaceutically acceptable carrier and/or excipient according to methods that can be readily carried out by those skilled in the art. In this case, the formulation may be in the form of a solution, suspension, or emulsion in an oil or aqueous medium, or in the form of a powder, granule, tablet, or capsule.

### Advantageous Effects

The feeder cells for NK cells according to the present invention express one or more of mbCD16, mbIL-12, and mbIL-18 together with HLA-E, thereby stimulating NK cells to enhance the activation and expansion of NK cells, and such NK cells can be used as a cell therapy product and as a pharmaceutical composition for the prevention or treatment of cancer or infectious diseases.

### Brief Description of Drawings

FIG. 1 shows the expression levels of HLA-E in K562 expressing HLA-E (K562-HLA-E), K562 expressing HLA-E and UL40F (K562-HLA-E UL40F), and K562 expressing HLA-E and UL40I (K562-HLA-E UL40I), which are feeder cells according to one embodiment of the present invention.
FIG. 2 shows the expression levels of mbCD16-1 or mbCD16-2 in K562 expressing HLA-E and mbCD16-1 (K562-HLA-E-mbCD16-1) and K562 expressing HLA-E and mbCD16-2 (K562-HLA-E-mbCD16-2), which are feeder cells according to one embodiment of the present invention.
FIG. 3 shows the expression levels of mbCD16-1 or mbCD16-2 in K562 expressing HLA-E and mbCD16-1 (K562-HLA-E UL40F-mbCD16-1) and K562 expressing HLA-E and mbCD16-2 (K562-HLA-E UL40F-mbCD16-2), which are feeder cells according to one embodiment of the present invention.
FIG. 4 shows the expression levels of mbCD16-1 or mbCD16-2 in K562 expressing HLA-E and mbCD16-1 (K562-HLA-E UL40I-mbCD16-1) and K562 expressing HLA-E and mbCD16-2 (K562-HLA-E UL40I-mbCD16-2), which are feeder cells according to one embodiment of the present invention.
FIG. 5 shows the expression levels of mbIL-12 and mbIL-18 in K562 expressing HLA-E, mbCD16-1, mbIL-12, and mbIL-18 (K562-HLA-E UL40F-mbCD16-1-mbIL12/18), which is a feeder cell according to one embodiment of the present invention.
FIG. 6 shows the gNK expression levels co-cultured with feeder cells according to one embodiment of the present invention, wherein A and B used PBMCs from different donors.
FIG. 7 shows the cytotoxic killing ability of gNK co-cultured with feeder cells according to one embodiment of the present invention.

### Detailed Description of Embodiments

Hereinafter, one or more embodiments will be described in more detail through examples. However, these examples are for illustrative purposes only to explain one or more embodiments, and the scope of the present invention is not limited to these examples.

### Example 1. Preparation of Feeder Cells

K562 (human myelogenous leukemia cell line) was obtained from the American Type Culture Collection (ATCC). The cells were cultured in RPMI 1640 medium supplemented with 10% heat-inactivated FBS (Gibco), penicillin 100 U/mL, and streptomycin 100 µg/mL (Invitrogen) at 37°C in a humidified 5% CO₂ incubator. K562 was then used to prepare feeder cells.

### 1-1. Preparation of HLA-E-Expressing Feeder Cells

K562 expressing HLA-E was prepared, and UL40F or UL40I, a CMV peptide, was additionally expressed to promote the expansion of NK cells.

First, the human-derived HLA-E gene (SEQ ID NO: 1), UL40F (SEQ ID NO: 2), or UL40I (SEQ ID NO: 3) was cloned into the lentiviral vector pCDH-CMV-EF1-GFP to prepare recombinant lentivirus production vectors Vector_HLA-E (SEQ ID NO: 4), Vector_HLA-E-UL40F (SEQ ID NO: 5), and Vector_HLA-E-UL40I (SEQ ID NO: 6). Subsequently, each of the prepared vectors was transfected into 293FT cells together with a packaging vector using lipofectamine 3000 (Invitrogen) for virus production. After a period of time, the medium was replaced with fresh medium and the cells were cultured for 48 hours, after which the virus-containing medium was collected. The collected medium was centrifuged at 500 × g for 10 minutes, and the pure virus-containing medium was separated using a 0.45 µm filter to produce lentiviruses expressing HLA-E, HLA-E + UL40F, or HLA-E + UL40I. Subsequently, 1 mL of each respective lentivirus was dissolved into 9 mL of medium containing K562 and added together with polybrene (8 µg/mL), and cell culture was carried out for 48 hours. Thereafter, infected cells were selected using fluorescence-activated cell sorting (FACS).

For flow cytometry, cell surface proteins and intracellular proteins were stained. NK cells and gNK cells were confirmed for purity by expression of CD3⁻CD56+ and CD56+FcRγ⁻/CD3⁻CD56+, respectively.

First, for cell surface protein staining, approximately 1 × 10⁶ cells were transferred to a 96-well U-bottom plate, centrifuged to remove the supernatant while retaining the cells. Each antibody for the surface protein to be analyzed was added at 1 µL each to 100 µL of FACS buffer (1% FBS in PBS): CD3 (APC-Cy7) (Invitrogen, 47-0036-42), CD56 (PE-Cy7) (Invitrogen, 25-0567-42), CD57 (APC) (Biolegend, 359610), and NKG2C (BV421) (BD, 748169). 100 µL of FACS buffer containing each antibody was added to the 96-well plate containing the cells, and staining was carried out for 15 minutes at 4°C in the dark. After washing with FACS buffer, the cells were centrifuged and the supernatant was removed, retaining only the cells.

For intracellular protein staining, the Fixation/Permeabilization Kit (BD, 554714) was used. 100 µL of 1× Perm/Wash buffer containing 1 µL of FcεRI (γ subunit-FITC) (Merck, FCABS400F) was added to the cells and stained for 30 minutes at 4°C in the dark. After washing with 1× Perm/Wash buffer, the cells were centrifuged and the supernatant was removed. 200 µL of 1× Perm/Wash buffer was added to the cells and flow cytometry was performed.

The feeder cells expressed reporter genes such as GFP and RFP and were not stained separately.

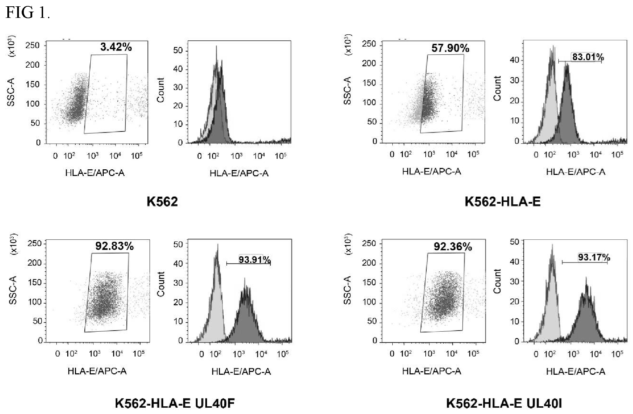

As a result, referring to FIG. 1, among the selected cells, K562 expressing HLA-E was designated 'K562-HLA-E', and K562 expressing HLA-E together with UL40F or UL40I was designated 'K562-HLA-E UL40F' and 'K562-HLA-E UL40I', respectively.

### 1-2. Preparation of HLA-E- and mbCD16-Expressing Feeder Cells

CD16 is a key factor promoting the selective expansion of gNK cells that are deficient in FcεRIγ among NK cells, and feeder cells expressing a membrane-bound form of anti-CD16 antibody (mbCD16-1 or mbCD16-2) together with HLA-E were prepared.

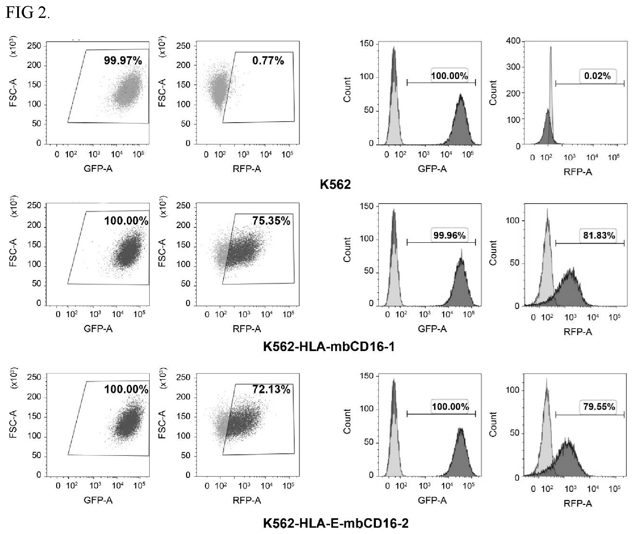

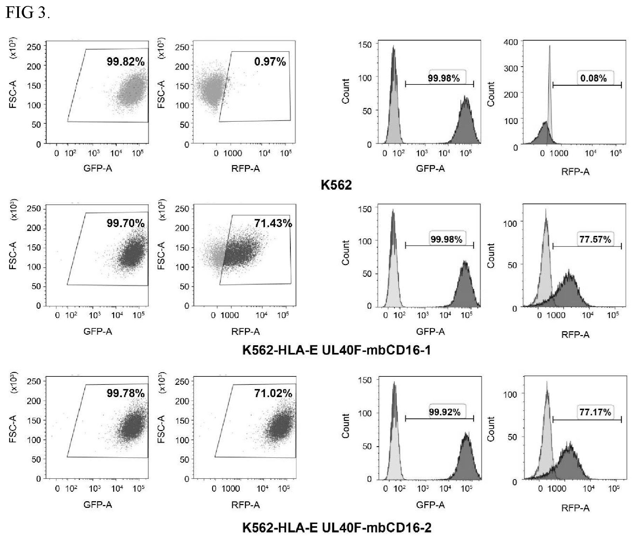

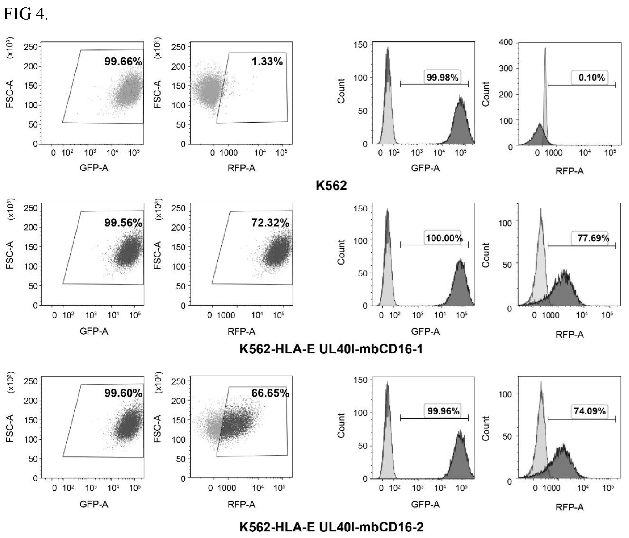

First, mbCD16-1 (SEQ ID NO: 7) or mbCD16-2 (SEQ ID NO: 8), which is the monoclonal antibody clone 3G8 of the anti-CD16 antibody, was cloned into the lentiviral vector pCDH-CMV-EF1-RFP to prepare recombinant lentivirus production vectors Vector_mbCD16-1 (SEQ ID NO: 9) and Vector_mbCD16-2 (SEQ ID NO: 10). Subsequently, each of the prepared vectors was transfected into 293FT cells together with a packaging vector using lipofectamine 3000 (Invitrogen) for virus production. After a period of time, the medium was replaced with fresh medium and the cells were cultured for 48 hours, after which the virus-containing medium was collected. The collected medium was centrifuged at 500 × g for 10 minutes, and the pure virus-containing medium was separated using a 0.45 µm filter to produce lentiviruses expressing mbCD16-1 or mbCD16-2. Subsequently, 1 mL of each respective lentivirus was dissolved into 9 mL of medium containing K562-HLA-E, K562-HLA-E UL40F, or K562-HLA-E UL40I prepared in Example 1-1 and added together with polybrene (8 µg/mL), and cell culture was carried out for 48 hours. Thereafter, infected cells were selected using flow cytometry. Referring to FIGS. 2 to 4, among the selected cells, K562 expressing HLA-E and mbCD16-1 was designated 'K562-HLA-E-mbCD16-1', 'K562-HLA-E UL40F-mbCD16-1', and 'K562-HLA-E UL40I-mbCD16-1'; and K562 expressing HLA-E and mbCD16-2 was designated 'K562-HLA-E-mbCD16-2', 'K562-HLA-E UL40F-mbCD16-2', and 'K562-HLA-E UL40I-mbCD16-2'.

### 1-3. Preparation of HLA-E-, mbCD16-, mbIL-12-, and mbIL-18-Expressing Feeder Cells

gNK cells have reduced expression of receptors for the cytokines IL-12 or IL-18, and thus responsiveness to IL-12 or IL-18 may be attenuated during NK cell expansion. Therefore, feeder cells expressing membrane-bound IL-12 and IL-18 (mbIL-12/18) together with HLA-E and mbCD16 were prepared to provide cytokine stimulation so as to supplement memory function during the expansion phase.

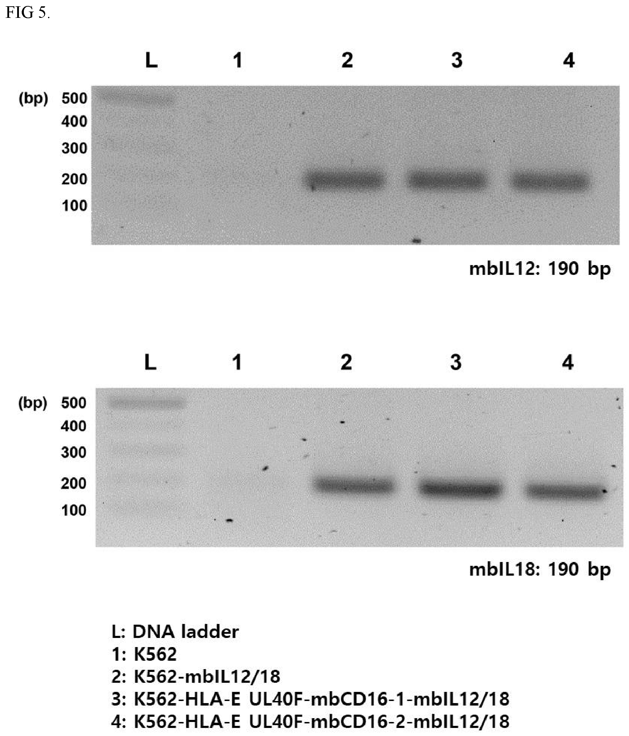

First, mbIL-12 (SEQ ID NO: 11) and mbIL-18 (SEQ ID NO: 12) were cloned into the lentiviral vector pCDH-CMV-EF1-GFP to prepare a recombinant lentivirus production vector Vector_mbIL12/18 (SEQ ID NO: 13). Subsequently, the prepared vector was transfected into 293FT cells together with a packaging vector using lipofectamine 3000 (Invitrogen) for virus production. After a period of time, the medium was replaced with fresh medium and the cells were cultured for 48 hours, after which the virus-containing medium was collected. The collected medium was centrifuged at 500 × g for 10 minutes, and the pure virus-containing medium was separated using a 0.45 µm filter to produce a lentivirus expressing mbIL-12 and mbIL-18. Subsequently, 1 mL of the lentivirus was dissolved into 9 mL of medium containing K562-HLA-E UL40F-mbCD16-1 prepared in Example 1-2 and added together with polybrene (8 µg/mL), and cell culture was carried out for 48 hours. Thereafter, infected cells were selected using flow cytometry, and the expression of mbIL-12 and mbIL-18 in the selected cells was confirmed using RT-qPCR (Quantitative reverse transcription PCR) (FIG. 5). Among the selected cells, K562 expressing HLA-E, mbCD16-1, mbIL-12, and mbIL-18 was designated 'K562-HLA-E UL40F-mbCD16-1-mbIL12/18'.

For PCR, the feeder cells were lysed to extract mRNA. Using 1 mg of the extracted mRNA, 1 mg of cDNA was synthesized, and mbIL-12 and mbIL-18 were amplified using this as a template. PCR was performed using the primers in Table 1 below at a temperature of 60°C for 30 cycles.

**[Table 1]**

| **Primer Name** | **Primer Sequence (5'-3')** | **SEQ ID NO** |
|---|---|---|
| mbIL18_F | GAA TGA CCA AGT TCT CTT CAT TGA CCA AG | 14 |
| mbIL18_R | GTT CTC ACA GGA GAG AGT TGA AAT TTT CTC | 15 |
| mbIL12_F | GAT GCC GTT CAC AAG CTC AAG | 16 |
| mbIL12_R | GAA CGC AGA ATG TCA GGG AGA AGT AG | 17 |

### Example 2. Phenotypic Characterization of NK Cells Expanded by Feeder Cells

Peripheral blood mononuclear cells (PBMCs) were isolated from healthy adult donors using Ficoll-Hypaque (d = 1.077, Lymphoprep^{™}; Axis-Shield, Oslo, Norway), and washed twice with PBS. The feeder cells of Example 1 were each prepared by irradiation with 100 Gy of gamma radiation.

For co-culture of feeder cells and PBMCs, the feeder cells (2.55 × 10⁵ cells/well) and PBMCs (5 × 10⁵ cells/well) of Example 1 were cultured in RPMI 1640 medium (containing 10% FBS, penicillin 100 U/mL, streptomycin 100 µg/mL, and L-glutamine 4 mmol/L) containing IL-2 10 U/mL, IL-12 10 U/mL, and IL-18 100 ng/mL in a 24-well plate. In co-culture with feeder cells expressing membrane-bound IL-12 and IL-18, IL-12 and IL-18 were not added. For re-stimulation on days 7 and 14, gamma-irradiated feeder cells were added. The medium was replaced every 2 to 3 days using RPMI 1640 medium containing IL-2 200 U/mL and IL-15 5 ng/mL, and the cells were cultured until day 21. The phenotypic characteristics of the expanded NK cells were confirmed by flow cytometry on culture days 7, 14, and 21.

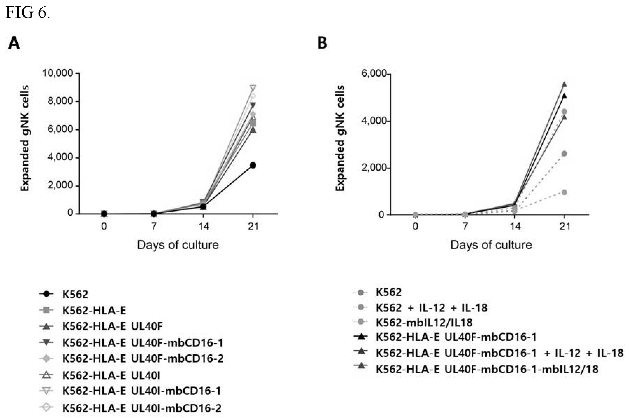

As a result, as shown in FIG. 6, when HLA-E was expressed in feeder cells, the expression level of gNK cells among expanded NK cells was higher than when HLA-E was not expressed, and the expression level of gNK cells further increased as mbCD16-1/2 was additionally expressed. In particular, when co-cultured with feeder cells expressing all of HLA-E, mbCD16-1, and mbIL-12/18, the expression level of gNK cells was highest.

### Example 3. ADCC Evaluation of NK Cells Expanded by Feeder Cells

gNK cells are deficient in FcεRIγ and therefore have lower cytotoxicity than conventional NK cells. However, when gNK cells bind to anti-CD16 antibody, the CD16 signal is strengthened, and thus the ADCC function is characteristically enhanced. Therefore, in order to evaluate the ADCC of gNK expanded by the feeder cells K562-HLA-E UL40F-mbCD16-1-mbIL12/18 of Example 1, cytotoxic killing ability was analyzed.

First, Raji cells were stained with 0.5 µM CFSE in FACS buffer for 10 minutes at 37°C and washed twice with RPMI medium. CFSE-stained Raji cells were coated with 10 µg/mL rituximab for 3 minutes at room temperature. gNK cells on culture day 14 and Raji cells were mixed in 96-well U-bottom plates at E:T ratios (2:1, 1:1, 0.5:1). The plate was centrifuged at 400 × g for 3 minutes and then cultured in a 5% CO₂ incubator for 4 hours. After culture, the cells were stained with 1 µg/mL propidium iodide and the cytotoxic killing ability was analyzed using flow cytometry.

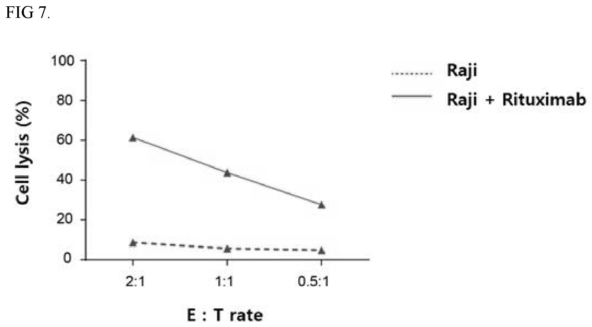

As a result, as shown in FIG. 7, gNK obtained on culture day 14 in co-culture with feeder cells K562-HLA-E UL40F-mbCD16-1-mbIL12/18 exhibited cytotoxic killing ability against the lymphoma cell line Raji, and when rituximab, an antibody targeting CD20, was added during co-culture, the ADCC of gNK was further increased.

The present invention has been described above with a focus on its preferred embodiments. A person having ordinary skill in the art to which the present invention pertains will understand that the present invention can be implemented in modified forms without departing from the essential characteristics of the present invention. Therefore, the disclosed embodiments should be considered from an illustrative point of view rather than a limiting point of view. The scope of the present invention is shown in the patent claims rather than the foregoing description, and all differences within the equivalent scope thereof should be construed as being included in the present invention.

## Claims

1. A feeder cell for activation and expansion of NK cells, which expresses: human leukocyte antigen E; and one or more selected from the group consisting of a membrane-bound anti-CD16 antibody, membrane-bound interleukin-12, and membrane-bound interleukin-18.

2. The feeder cell according to claim 1, wherein the feeder cell is selected from the group consisting of K562, RPMI8866, ARH77, EBV_LCL, 721.221, and HFWT cells.

3. The feeder cell according to claim 1, wherein the membrane-bound anti-CD16 antibody is one or more selected from the group consisting of monoclonal antibodies, polyclonal antibodies, recombinant antibodies, and fragments thereof.

4. The feeder cell according to claim 3, wherein the monoclonal antibody is one or more selected from the group consisting of CB16, 3G8, B73.1, and MEM-154.

5. The feeder cell according to claim 1, wherein the feeder cell expresses human leukocyte antigen E, a membrane-bound anti-CD16 antibody, membrane-bound interleukin-12, and membrane-bound interleukin-18.

6. A composition for activation and expansion of NK cells, comprising the feeder cell of any one of claims 1 to 5.

7. The composition according to claim 6, wherein the composition further comprises a cytokine.

8. A method for activation and expansion of NK cells, comprising the step of contacting NK cells with the feeder cell of claim 1 or the composition of claim 6.

9. The method according to claim 8, wherein the NK cells are isolated from a blood sample.

10. The method according to claim 8, wherein the step is co-culturing NK cells and feeder cells.

11. The method according to claim 10, wherein the feeder cells are irradiated with 50 Gy to 300 Gy of radiation.

12. The method according to claim 10, wherein the co-culture involves culturing 0.1 to 10 × 10⁶ feeder cells per 1 × 10⁶ NK cells.

13. The method according to claim 10, wherein the co-culture is performed in the presence of a cytokine.

14. The method according to claim 13, wherein the cytokine is one or more selected from the group consisting of IL-1, IL-2, IL-3, IL-4, IL-5, IL-6, IL-7, IL-8 (CXCL8), IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-35, and IL-36.

15. A method for assaying NK cell activity, comprising steps of: i) contacting NK cells with the feeder cell of claim 1 or the composition of claim 6 to activate the NK cells; and ii) analyzing the degree of activation of the activated NK cells.

16. The method according to claim 15, wherein the degree of activation of the NK cells in step ii) is measured as one or more selected from the group consisting of degranulation activity, cytotoxicity activity, and cytokines secreted upon NK cell stimulation.

17. A method for providing information for diagnosis of NK cell-related diseases, comprising steps of: i) contacting NK cells with the feeder cell of claim 1 or the composition of claim 6 to activate the NK cells; and ii) analyzing the degree of activation of the activated NK cells.

18. The method according to claim 17, wherein the degree of activation of the NK cells in step ii) is measured as one or more selected from the group consisting of degranulation activity, cytotoxicity activity, and cytokines secreted upon NK cell stimulation.

19. NK cells produced by the method for activation and expansion of NK cells of claim 8.

20. A cell therapy product comprising the NK cells of claim 19 as an active ingredient.

21. A pharmaceutical composition for the prevention or treatment of cancer or infectious diseases, comprising the NK cells of claim 19 as an active ingredient.
